# EUROPEAN PATENT SPECIFICATION

(11) **EP 4 450 040 B1**
(45) Date of publication and mention of the grant of the patent: **04.11.2026**
(21) Application number: 23185954.7
(22) Date of filing: 17.07.2023
(51) Int. Cl.: A61F 13/531, A61F 13/532

(54) **ABSORBENT ARTICLE WITH CHANNEL(S)**
SAUGFÄHIGER ARTIKEL MIT KANAL(EN)
ARTICLE ABSORBANT À CANAUX

(30) Priority: 20.04.2023 EP 23168863; 20.04.2023 EP 23168880; 20.04.2023 EP 23168909; 11.07.2023 EP 23184685; 12.07.2023 EP 23185001; 12.07.2023 EP 23185021
(43) Date of publication of application: 23.10.2024
(60) Divisional application: 26176727.1 / 4 778 499
(73) Proprietor: Ontex BV, 9255 Buggenhout (BE); Ontex Group NV, 9320 Erembodegem (BE)
(72) Inventor: WEBER, Ainas, 53474 Bad Neuenahr-Ahrweiler (DE)
(74) Representative: Cobbaert, Dries

(56) References cited:
- EP-A1- 3 403 627
- EP-A1- 4 079 274
- US-A1- 2020 078 232

## Description

### TECHNICAL FIELD

The disclosure relates to absorbent articles such as disposable absorbent articles, preferably selected from the group consisting of diapers (whether for baby, youth, or adults), pants (whether for baby, youth or adults), briefs, pantyliners, slips, sanitary napkins or towels, sanitary napkins for light incontinence, and the like; preferably selected from diapers and pants.

### BACKGROUND

The disposable absorbent articles are articles which are used in proximity with the skin of a wearer to absorb and retain body exudates; they are typically constituted by an upper liquid-pervious topsheet, a bottom liquid impervious backsheet and an absorbent core disposed between them. The absorbent core of this kind of articles is generally composed of fibers of absorbent material and/or particles of superabsorbent material; its main functions are to absorb and to retain liquid or semi-liquid exudates from the human body that penetrate through the upper layer of the article. For the absorbent core to be effective, it desirably has a good integrity, that is to say, the absorbent core must maintain its structure and not break or collapse either in a dry or in a wet state. If the absorbent core breaks, its absorption and retention capacity as well as its ability to distributing liquids may be compromised and further increasing the risk of leakage, agglomerations of absorbent material and discomfort to the wearer. Multiple attempts have been made to prevent or minimize these shortcomings such as the use of nets, adhesives, core wraps, embossing and others.

Some examples that attempt to improve the integrity of the absorbent cores, include for example US Pat. No. 8, 198, 506 and US Pat No. 7,718,021 (Kimberly Clark Worldwide Inc.) which relate to a stabilized absorbent composite web and the method for producing it; said absorbent composite comprises a first sheet joined to a second sheet through a plurality of holes formed in the absorbent core. These patents are fully focused on improving the integrity of the absorbent core sacrificing the ability to absorb liquids more quickly as well as the ability to distribute them more evenly and efficiently through the absorbent core.

Other examples focus in improving the distribution of fluids in the absorbent core using channels. Articles comprising channelled cores are becoming more and more sought after in the market in view of their improved liquid distribution properties and reduction in amount of absorbent material used. Examples are described in the literature such as EP 3 342 386 A1, EP 3 692 963 A1, EP 3 542 766 A1, EP 3 738 562 A1 and the like. The preferred means of creating such channels is consistently by bonding directly upper and lower layers of an enveloping core wrap sheet in such a way to create channel-forming areas that are substantially free of absorbent material.

Other examples relate to absorbent cores free of cellulose fibers aiming at improving the containment and retention capacity of the core, as for example US20150342796A1 from Procter & Gamble which refers to an absorbent core comprising a core wrap enclosing superabsorbent particles: the superabsorbent particles form a pattern of dot-shape discrete areas which can be oriented longitudinally or transversely; these areas are separated from each other by areas substantially free of material and could also contain channels free of absorbent material.

Other examples relate to absorbent cores using zones having different basis weight and density within the core. Depending the way these zones are disposed, they are used for different purposes. For example, EP3238676B1 (Procter & Gamble) describes an absorbent core with first and second longitudinal channels free of absorbent material; the core may further comprise one or more transverse oriented folding lines in which the basis weight of absorbent material reaches a minimum relative to the immediately adjacent regions. These zones with lower basis weight are used for facilitating the folding and as they reach the longitudinal sides of the core.

Other attempts to overcome the above mentioned shortcomings are described for example in WO2021/123221 A1 that relates to a disposable absorbent article comprising a liquid-pervious topsheet, a liquid-impervious backsheet and an absorbent assembly between the topsheet and the backsheet. The absorbent assembly comprises an absorbent core, an upper core wrap and a lower core wrap. The absorbent core comprises at least two cavities free of absorbent material wherein the upper core wrap and the lower core wrap are permanently attached to each other, said cavities being interconnected via at least one connecting line comprising absorbent material. The absorbent core having a first basis weight in the zone out of the cavities and the connecting lines and a second basis weight in the connecting lines, the first basis weight being greater than the second basis weight. in the cavities.

EP4079274 relates to absorbent hygiene articles having a substantially continuous channel and a wetness indicator which can give the presence of a liquid bodily exudate in the article. The continuously extending wetness indicator, as seen from the garment-facing side of the article, is coincident with the longitudinal center line (LCL) of the article and comprises an overlapping portion superposed with at least part of the longitudinally extending channel central section, and an exposed portion superposed with absorbent material at least partially surrounded by channel sections closer to the front transverse edge of the article.

There however still remains a need for absorbent articles that have excellent core integrity whilst maintaining fast liquid distribution and being simple and cost-effective to manufacture.

### SUMMARY

The invention is as defined in the claims.

In a first aspect the disclosure relates to an absorbent article comprising a topsheet, a backsheet and an absorbent core sandwiched between said topsheet and backsheet and comprising a first longitudinal half and a second longitudinal half separated by a longitudinal centerline (y); said core comprising absorbent material that is substantially enclosed by a core wrap; wherein said core wrap comprises a top core wrap layer and a bottom core wrap layer; said core comprising one or more channels flanked by absorbent material that is positioned at a first absorbent material deposition zone. At least one or the channels, preferably each said channel, has a meandering shape extending along a longitudinal direction (Ld) running substantially parallel to a longitudinal centerline (y) of said absorbent core and comprising a plurality of turning points; each of said turning points forming an apex; wherein the plurality of turning points comprises at least a first turning point and at least a second turning point; and wherein said at least first turning point is offset from said at least second turning point such that an imaginary line running parallel to the longitudinal centerline (y) crosses the apex of the at least first turning point and not the apex of the at least second point or vice versa.

In a second aspect the disclosure related to an array of packages. The array comprising at least a first package comprising a first plurality of absorbent articles and at least a second package comprising a second plurality of absorbent articles; wherein the first and second plurality of absorbent articles differ in terms of one or more characteristics selected from: average apex-to-centerline distance in the front portion, average apex-to-centerline distance in the crotch portion, average apex-to-centerline distance in the rear portion.

### BRIEF DESCRIPTION OF THE FIGURES

**FIG.** 1 is a plan view representation of an article according to an embodiment of the present disclosure.
**FIG.** 2 is a plan view representation of an article according to an embodiment of the present disclosure.
**FIG.** 3 is a plan view representation of an absorbent core according to an embodiment of the present disclosure, showing an example of a meandering channel.
**FIG.** 4 is a plan view representation of an absorbent core according to an embodiment of the present disclosure, showing an example of a meandering channel with a straight section.
**FIG.** 5 is a plan view representation of an absorbent core according to an embodiment of the present disclosure, showing an examples of a meandering channel with a loop.
**FIG.** 6 is a plan view representation of an exemplary striped bonding pattern.
**FIG.** 7 is a plan view representation of an exemplary striped bonding pattern.
**FIG.** 8 is a plan view representation of an exemplary striped bonding pattern.
**FIG.** 9 is a plan view representation of an exemplary striped bonding pattern.

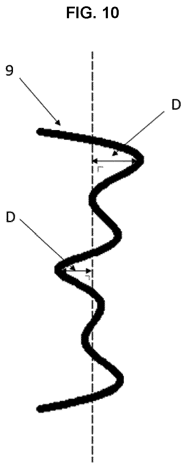

**FIG.** 10 is a plan view representation of the apex-to-centerline distance.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" or "substantially" as used herein referring to a measurable value such as a parameter, an amount, a temporal duration, and the like, is meant to encompass variations of +/-40% or less, or +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" or "substantially" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

By "channels", it is meant that the structure referred to (e.g. the absorbent core) comprises recessed regions forming visible conduits or passages typically extending along the longitudinal axis of the core and having a depth in a direction perpendicular to said longitudinal axis. By "visible" it is herein intended clearly visible by naked eye and typically that the channels have a width generally greater than 1mm, preferably from 5mm to 50mm, more preferably from 8mm to 40mm, more preferably from 10mm to 30mm, even more preferably from greater than 10mm to less than 25mm.

By "interconnected", it is meant that the structure referred to (e.g. the channels) forms a substantially continuous path such as from a first end of a channel to a second end of the same channel.

By "substantially", it is meant at least the majority of the structure referred to. For example, with reference to interconnected channels, "substantially interconnected" means that the majority of the channel is interconnected and generally wherein a direct and continuous path can be traced by starting from one end of the channel towards another end of the channel, said ends (also referred to herein as terminal positions) being distal to each other in a width direction of the core and proximal to a portion of the perimeter of the core, preferably the sides thereof.

"Front portion" as used herein refers to the portion of the absorbent core lying most proximal to the front product edge. The front portion has a length of 1/3 of the total length of the absorbent core, measured in flat and fully extended state.

"Rear portion" as used herein refers to the portion of the absorbent core lying most distal to the front product edge. The rear portion has a length of 1/3 of the total length of the absorbent core, measured in flat and fully extended state.

"Crotch portion" as used herein refers to the portion of the absorbent core lying between the front portion and the rear portion of the absorbent core. The crotch portion has a length of 1/3 of the total length of the absorbent core, measured in flat and fully extended state.

The expression "% by weight" or "%wt" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

The term "meandering" as used herein refers to following a winding or intricate course.

The terms "nonwoven", nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The term "disposable" refers to absorbent articles and/or inserts that generally are not intended to be laundered or otherwise restored or reused as absorbent articles, i.e., they are intended to be discarded after a single use and, preferably, to be recycled, composted or otherwise disposed of in an environmentally compatible manner.

The term "disposed" is used to mean that an element(s) is formed (joined and positioned) in a particular place or position as a, preferably unitary, structure with other elements or as a separate element joined to another element.

The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.

The term "joined" refers to configurations whereby an element is directly secured to another element by attaching the element directly to the other element, and configurations whereby an element is indirectly secured to another element by attaching the element to intermediate member(s) which in turn are attached to the other element.

The term "lateral" or "transverse" refers to a direction running at a 90 degree angle to the longitudinal direction and when combined with the term "substantially" includes directions within ±45° of the lateral direction.

The term "longitudinal" refers to a direction running parallel to the maximum linear dimension of the article and when combined with the term "substantially" includes directions within ±45° of the longitudinal direction.

Embodiments of the articles according to the disclosure will now be described. It is understood that technical features described in one or more embodiments maybe combined with one or more other embodiments without departing from the intention of the disclosure and without generalization therefrom, especially when such combinations are explicitly or implicitly inferred.

### THE ABSORBENT ARTICLE

The article (1) herein may be disposable and selected from the group consisting of diapers, pants, briefs, pantyliners, slips, sanitary napkins and towels, and combinations thereof, preferably diapers or pants (for baby, youth, and/or adult subjects).

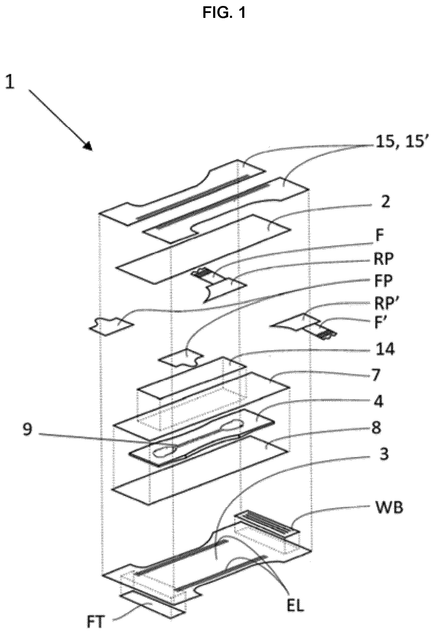

Absorbent articles (1) described herein, and as exemplified in Figs. 1-2, comprise: a topsheet (2); a backsheet (3); and an absorbent core (4) sandwiched between said topsheet (2) and backsheet (3); said core (4) typically comprises absorbent material (5) that is substantially enclosed by a core wrap (6), wherein said core wrap (6) may comprise a top core wrap layer (7) and a bottom core wrap layer (8). The absorbent core (4) has an outer perimeter (10) formed by two opposed lateral edges (11, 11') connecting two opposed transversal ends (12, 12') of said core (4).

The absorbent core (4) comprises a first longitudinal half, being the left longitudinal half (L), and a second longitudinal half, being the right longitudinal half (R), running along a length of said core (4) and being adjacent to each other with the longitudinal centerline (y) being interposed therebetween. Furthermore, the absorbent core (4) comprises a front portion (F), a crotch portion (C) and a rear portion (R).

In an embodiment, the article (1) further comprises a liquid distribution layer (14) positioned between the topsheet (2) and the absorbent core (4), the said layer may be any suitable nonwoven layer (including multi-layer laminates). Preferably the liquid distribution layer is a nonwoven layer selected from the group consisting of a spunbond nonwoven, carded nonwoven, spunlace nonwoven, and combinations thereof. The basis weight of the liquid distribution layer (14) is preferably from 15 gsm (g/m2) to 45 gsm (g/m2), preferably from 18 gsm (g/m2) to 40 gsm (g/m2), even more preferably from 20 gsm (g/m2) to 38 gsm (g/m2).

Preferably, the absorbent material (5) comprises superabsorbent polymer particles and/or cellulose fibers. The superabsorbent polymer particles may be comprised in an amount of from 50%wt to 100%wt, preferably from 60%wt to 90%wt, preferably from 70%wt to 85%wt, even more preferably from 75%wt to 85%wt, by total weight of the absorbent material. Having some cellulose fiber content may be advantageous in providing core integrity whilst limiting the amount of adhesive used as well as aid capillary flow effects described herein.

The article (1), as exemplified in Fig. 1, herein may further comprise oppositely disposed lateral cuffs (15, 15') that generally act as longitudinal barriers to liquid leakage. The cuffs typically comprise a hydrophobic nonwoven layer and one or more elastics proximal to an apex thereof to form stand-up gathers. For clarity of the drawings, only one or two elastic strands are shown for each cuff, but typically each cuff may comprise from 1 to 4 elastic strands. The absorbent article may also comprise other typical components such as a back elastic waist feature (WB), a front elastic waist feature, frontal tape or landing zone (FT), leg elastics (EL) transverse barrier cuffs, front panels (FP), rear panels (RP, RP') that may be elastic and may comprise fasteners (F, F') for joining to the frontal tape, a wetness indicator that changes colour when contacted with urine, etc.

### CHANNEL(S)

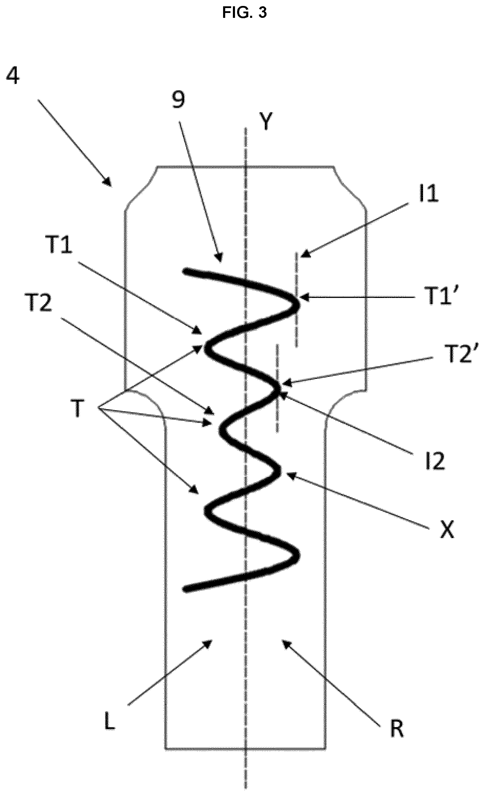

The absorbent core (4) of present invention comprises one or more channels (9) flanked by absorbent material (5). At least one of the channels (9), preferably each said channel, has a meandering shape extending along a longitudinal direction (Ld) running substantially parallel to a longitudinal centerline (y) of said absorbent core (4). The meandering shape comprises a plurality of turning points (T), as exemplified in Fig. 3, wherein each of said turning points (T) forms an apex (X). The apex (X) is the highest point or top of a shape or object. In this case, the apex (X) of said turning points (T) is the point along each turning point where the tangent is equal to 0. The plurality of turning points (T) comprises at least a first turning point (T1) and at least a second turning point (T2). The at least first turning point (T1) is offset from said at least second turning point (T2) such that an imaginary line (I1, I2) running parallel to the longitudinal centerline (y) crosses the apex (X) of the at least first turning point (T1) and not the apex (X) of the at least second turning point (T2) or vice versa. Advantageously this provides for an improved usage of the total absorbent capacity of the absorbent core (4) and for an improved liquid distribution.

In an embodiment, at least a left first turning point (T1) is located in the first longitudinal half, being the left longitudinal half (L), and at least a left second turning point (T2) is located in the first longitudinal half. An imaginary line (I1, I2) running parallel to the longitudinal centerline (y) crosses the apex (X) of the at least left first turning point (T1) and not the apex (X) of the at least left second turning point (T2) or vice versa.

In an embodiment, at least a right first turning point (T1') is located in the second longitudinal half, being the right longitudinal half (R), and at least a right second turning point (T2') is located in the second longitudinal half (R). An imaginary line (I1, I2) running parallel to the longitudinal centerline (y) crosses the apex (X) of the at least right first turning point (T1) and not the apex (X) of the at least right second turning (T2) point or vice versa.

In a preferred embodiment, as exemplified in Fig. 3, at least a left first turning point (T1) and at least a left second turning point (T2) are located in the first longitudinal half (L) and at least a right first turning point (T1') and at least a right second turning point (T2') are located in the second longitudinal half (R). An imaginary line (I1, I2) running parallel to the longitudinal centerline (y) crosses the apex (X) of the at least left first turning point (T1) and not the apex(X) of the at least left second turning point (T2) or vice versa in the first longitudinal half (L) and an imaginary line (I1, I2) running parallel to the longitudinal centerline (y) crosses the apex (X) of the at least right first turning point (T1') and not the apex (X) of the at least right second turning point (T2') or vice vera in the second longitudinal half (R).

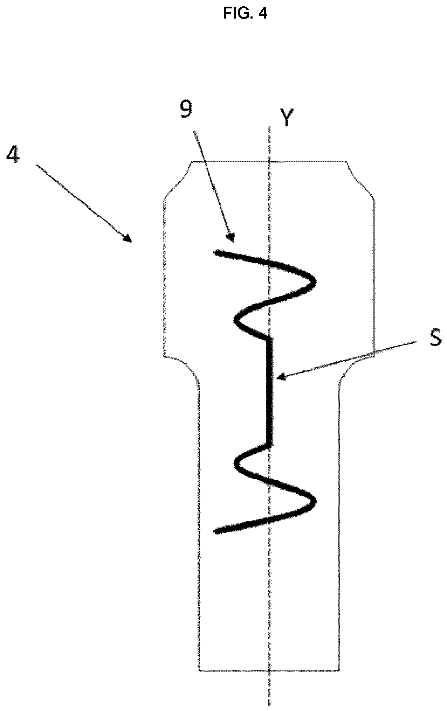

In an embodiment, as exemplified in Fig. 4, one or more channels (9) comprise a meandering shape containing a straight section (S) running substantially parallel to a longitudinal centerline (y) of said absorbent core (4). The straight section (S) is preferably longitudinally centered on a miction point which is at a distance from the front transverse edge (12) along the longitudinal centerline (y) of between 30% and 55%, more preferably between 35% and 53%, even more preferably between 40% and 50%, of the absorbent core length. This advantageously allows the miction point positioning to correspond to the central section (S), so that liquid distribution towards front and back may quickly start. In this same embodiment, the straight section (S) has a length of more than 2%, more preferably between 2 and 25% or between 4 and 25%, more preferably between 5 and 20%, even more preferably between 6 and 15%, most preferably between 7 and 12% of the absorbent core length. If the central section (S) is too short, the absorption time may increase and rewet, to some extent, also may increase. When the central section (S) is too long, both absorption time and rewet may greatly increase.

In an embodiment, at least one or more channels (9), preferably having a meandering shape, extend into the front portion (F) or rear portion (B). Alternatively, at least one or more channels (9), preferably having a meandering shape, extend into the front (F) and rear (B) portion. Advantageously, this allows for adequate distribution of liquid towards the front (F) and/or rear portion (B) of the absorbent core (4) which in turn leads to improved use of the total absorbent capacity of the absorbent core (4).

In a preferred embodiment, the average apex-to-centerline distance (D) of the one or more channels (9) is lower in the crotch portion (C) than in the front (F) and rear portion (B). Advantageously, this allows for a quick distribution of the liquid towards the front portion (F) and rear portion (B) of the absorbent core (4) in order to better use the total absorbent capacity of the absorbent core (4). The higher average apex-to-centerline distance (D) in the front (F) and rear portion (B) allows for better distribution in width direction and at the same time reduces the risk of leakage towards the first and second transverse edges (12, 12') of the absorbent core (4).

In an embodiment, the average apex-to-centerline distance (D) is lower in the front portion (F) or rear portion (B) than in the crotch portion (C). Advantageously, this allows to more efficiently utilize the total absorbent capacity in the crotch portion (C) while minimizing the risk of leakage in front (F) or rear portions (B) towards the first and second longitudinal edges (11,11') of the absorbent core (4).

In an embodiment, the average apex-to-centerline distance (D) is lower in the front portion (F) and rear portion (B) than in the crotch portion (C). Advantageously, this allows to more efficiently utilize the total absorbent capacity in the crotch portion (C) while minimizing the risk of leakage in front (F) and rear portions (B) towards the first and second longitudinal edges (11, 11') of the absorbent core (4).

In an embodiment, the average apex-to-centerline distance (D) is the same in the crotch portion (C) as in the front (F) and rear portions (B). Advantageously, this allows to more efficiently utilize the total absorbent capacity and have the same distribution properties in all portions. Without being bound by theory, this might be especially beneficial when having a rectangularly shaped absorbent core (4).

In a preferred embodiment, the average apex-to-centerline distance (D) is lower in the crotch potion (C) as in the front (F) and rear portions (B). Advantageously, this provides for quick distribution of liquid while maximizing the usage of the total absorbent capacity of the absorbent core (4).

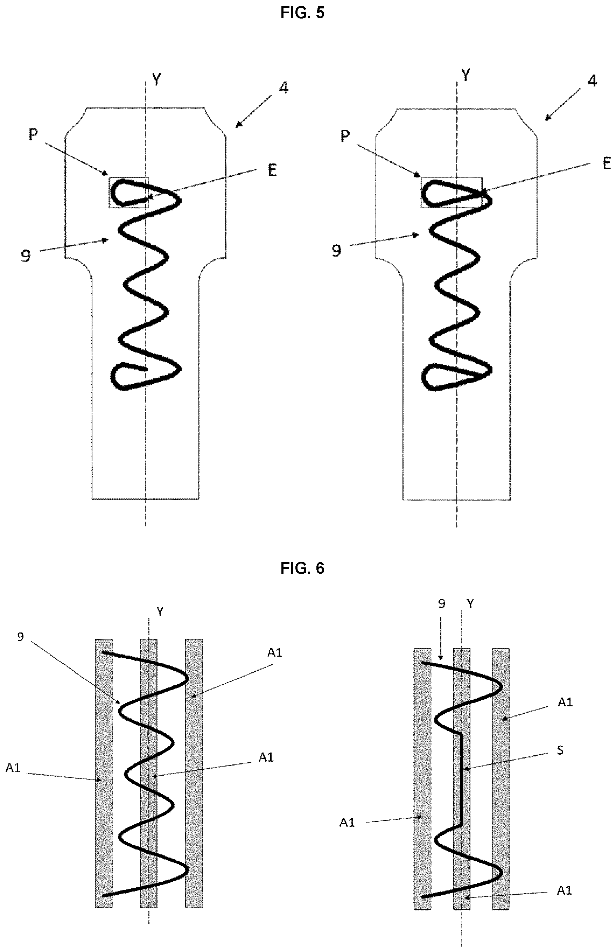

In an embodiment, as exemplified in Fig. 5, the absorbent core (4) comprises at least one meandering channel (9) forming a loop (P) in the front (F) and/or rear portion (B) of the core (4) wherein said loop (P) is formed by connecting at least one of the ends (E) of the at least one meandering channel (9) to itself. Advantageously, this reduces the chance of leakage by avoiding the presence of an end (E) of the channel (9) being directed towards one of the longitudinal edges (11, 11') of the absorbent core (4).

In an alternative embodiment, as exemplified in Fig. 5, the absorbent core (4) comprises at least one meandering channel (9) forming a loop (P) in the front (F) and/or rear portion (B) of the core (4) wherein at least one of the ends (E) of the at least one meandering channel (9) is not connected to said meandering channel (9). Advantageously, this reduces the chance of leakage by avoiding the presence of an end (E) of the channel being directed towards one of the longitudinal edges (11, 11') of the absorbent core (4).

In an embodiment, the number of turning points is the same in the crotch portion (C) as in the front (F) and rear portions (B). Advantageously, this allows to more efficiently utilize the total absorbent capacity and have the same distribution properties in all portions. Without being bound by theory, this might be especially beneficial when having a rectangularly shaped absorbent core (4).

In an embodiment, the number of turning points (T) in the crotch portion (C) is less than the number of turning points (T) in the front (F) and/or rear portion (B). Advantageously, this provides for quick distribution of liquid while maximizing the usage of the total absorbent capacity of the absorbent core (4).

Preferably, the at least one or more channels (9) do not extend to a perimeter (10) of the core (4) and are generally arranged such that the entire channel(s) (9) is substantially continuously surrounded by the absorbent material of the absorbent-material-deposition zone (Z_{amd}). Advantageously, this avoids liquid running out of the absorbent core (4).

The total length of the channel(s) (9) herein may be from 10% to 95%, preferably from 12% to 90%, more preferably from 15% to 85%, even more preferably from 20% to 80%, of a length of the absorbent core (4) generally said lengths taken along a longitudinal direction running substantially parallel to the longitudinal centerline (y). The one or more channels (9) thus have a length along a longitudinal axis (y) that is less than a length of the absorbent core (4) extending along said axis (y) such that the one or more channels (9) do not extend to the perimeter of the core (4). Advantageously this aids to limit risk of undesirable leakage from the transverse edges (12, 12') of the core (4) and article (1).

### BONDING PATTERN

The absorbent cores (4) herein comprise an absorbent-material-deposition zone (Z_{amd}) comprising a first basis weight of absorbent material (5) and are substantially enclosed by a core wrap (6), wherein said core wrap (6) may comprise a top core wrap layer (7) and a bottom core wrap layer (8). The top (7) and bottom layer (8) of the core wrap layer (6) are typically joined along one or more continuous or discontinuous attachment areas. The one or more continuous or discontinuous attachment areas may form one or more channels (9) typically together with or in conjunction with other structures (e.g. unbonded regions) as will be described in more detail herein below. The one or more channels (9), generally, are substantially free of absorbent material.

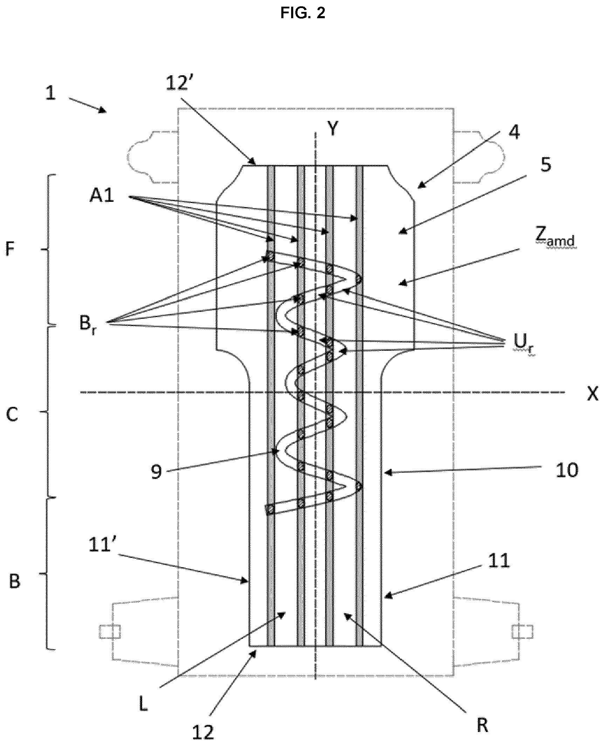

In an embodiment, as exemplified in Fig. 2, the top core wrap layer (7) and a bottom core wrap layer (8) are joined together at a plurality of discrete bonding regions (Bᵣ) substantially free of absorbent material (5) (for example the amount of absorbent material in the bonding region is less than about 5 gsm, preferably less than about 3 gsm, even more preferably less than about 2 gsm, most preferably about 0 gsm). The bonding may be carried out by the use of adhesive or by mechanically bonding the layers together such as ultrasonic bonding, thermal bonding, combinations thereof and the like. Advantageously this allows to ensure good core integrity as well as maximised surface area coverage of liquid distribution via mass flow over an initial wetting period.

In an embodiment, the discrete bonding regions (Bᵣ) are separated by at least a portion of said absorbent-material-deposition zone (Z_{amd}) along a longitudinal direction (Ld) running substantially parallel to a longitudinal centerline (y) of said absorbent core (4).

Preferably, the discrete bonding regions (Bᵣ) have an aspect ratio of from 0.4 to 3.0, preferably from 0.5 to 2.5, even more preferably of about 1. Advantageously this allows to limit the size of restriction areas continuously formed along top/bottom core wrap joints. Moreover, advantageously, this may permit each discrete bonding regions (Bᵣ) to not form a channel hence maximizing the available space for liquid absorption (yet together with unfastened regions yet being able to forming channel(s), in at least dry state and a first wetting state, to still allow for an initial liquid distribution by mass flow as described in more detain within embodiments herein).

The one or more bonding regions (Bᵣ) may comprise permanent and/or temporary attachments. Typically, permanent attachments being arranged to remain substantially integral upon wetting and temporary attachments being arranged to release upon wetting. By wetting being intended exposing the said attachments to liquid for at least about 20 seconds, preferably at least about 30 seconds, more preferably at least about 50 seconds (typically at room conditions and with a liquid being at a body temperature of about 37 °C, the liquid may be a saline solution having 0.9% NaCl). For example, permanent attachments may comprise adhesive that is substantially liquid insoluble and temporary attachments may comprise adhesive that is substantially liquid soluble, or by other joining means e.g. by selective application of lower joining pressure, or contaminating the areas to be bonded with a dusting layer of absorbent, or the like generally forming a weaker attachment so that upon swelling of the absorbent material the force generated pulling the said layers apart is greater than the joining force provided by the joining means. Other examples of permanent attachments include mechanical bonding such as ultrasonic bonding, thermal and pressure bonding, and the like.

The one or more bonding regions (Bᵣ) may comprise permanent and temporary attachments typically alternatingly disposed such that each permanent attachment is adjacent to, preferably interposed between, said temporary attachment(s); or vice versa. Advantageously this allows the formation of intermediate capillary acceleration zones allowing liquid to be transferred by capillary action especially during a second time period of wetting as described herein. Moreover, such temporary attachments permit to reduce the overall size (e.g. area and/or length) of the bonding regions especially during said second time period making a larger surface of the core available for wicking of liquid to maximise absorbent core usage.

Preferably, as for example shown in Fig. 2, the discrete bonding regions (Bᵣ) are positioned outboard of a longitudinal centreline (y) of said absorbent core (4) such that said longitudinal centreline (y) is not substantially overlapped by said discrete bonding regions (Bᵣ).

Advantageously this may allow maximising absorption capacity and free-swelling mechanisms in the central region of the core.

In a further embodiment, as shown in Fig. 2, the absorbent cores (4) herein comprise one or more unfastened regions (Uᵣ) comprising a second basis weight of absorbent material (5), and wherein the first basis weight may be greater than the second basis weight. The discrete bonding regions (Bᵣ) and the one or more unfastened regions (Uᵣ) may be substantially connected to each other (for example when viewed in a planar direction and/or collectively form a continuous shape or channel typically at least in dry state). Advantageously, this permits volume expansion within cavities formed in the unfastened regions to accommodate neighbouring absorbent material as it swells whilst minimising the size of restriction areas formed at top/bottom core wrap joints. I may also further aid to reduce cost by permitting a reduction in amount of absorbent material yet retain a larger area for swelling capacity in wet state.

The one or more unfastened regions (Ur) may be substantially free of absorbent material (typically in dry state, generally prior to cavity filling by absorbent material during wetting/swelling) generally meaning that the second basis weight may be less than about 5gsm, preferably less than about 3 gsm, even more preferably less than about 2gsm, most preferably about (i.e. substantially) 0 gsm. Advantageously, although no connection of top and bottom core wrap layers is present in such region the substantial absence of absorbent material (at least during the early wetting stages) in such region facilitates fluid transfer by mass flow between connecting bonding regions. Such may further permit maintaining the desired fluid distribution characteristics as well as accommodating absorbent material swelling whilst retaining core integrity in an effective manner.

Although the unfastened regions (Ur) may not be permanently (or physically/chemically) joined together, such regions may still comprise top and bottom core wrap layers (7, 8) in close proximity to each other (e.g. the layers may be adjacent or immediately adjacent to each other). This may be achieved for example by calendaring or otherwise compressing the absorbent core laminate after the step of enclosing the absorbent material therein as for example described in more detail herein below.

At least portions of the unfastened regions (Uᵣ) may be separated by the absorbent-material-deposition zone (Z_{amd}) along at least an axis substantially parallel to the longitudinal centreline (y) and/or at least some of the discrete bonding regions (Bᵣ) may be separated by the absorbent-material-deposition zone (Z_{amd}) along at least an axis substantially parallel to the longitudinal centreline (y). Such arrangement may permit effective liquid distribution by wicking in areas of the core between unfastened regions.

The one or more bonding regions (Bᵣ) are generally in the form of discrete bonding regions, and preferably wherein the article comprises a plurality of unfastened regions (Uᵣ) separated by discrete bonding regions (Bᵣ) generally meaning that consecutive unfastened regions (Uᵣ) are separated from each other by discrete bonding regions (Bᵣ); preferably wherein said plurality of unfastened regions (Uᵣ) separated by discrete bonding regions (Bᵣ) are positioned in a front half and/or rear half of the absorbent core (4).

In an embodiment, the one or more bonding regions (Bᵣ) and the one or more unfastened regions (Uᵣ) are substantially connected to each other to form one or more channel(s) (9) extending along a longitudinal direction (L_{d}) running substantially parallel to a longitudinal centreline (y) of said absorbent core (4) wherein said one or more channel(s) and/or circumscribed by absorbent material (5) of the absorbent-material-deposition zone (Z_{amd}). The one or more unfastened regions (Uᵣ) are substantially free of absorbent material such that a ratio of second basis weight to first basis weight (i.e. second basis weight / first basis weight) is less than 0.25, preferably less than 0.2, even more preferably from, or between, 0 and 0.15. Preferably the one or more channel(s) are continuous generally such that the one or more bonding regions (Bᵣ) and the one or more unfastened regions (Uᵣ) are directly connected to each other. Advantageously liquid mass flow through the one or more channel(s) is not substantially obstructed by absorbent material and a cavity to accommodate absorbent material during wetting may be formed directly within the unfastened regions between bonded regions for efficient absorption.

The absorbent article (1) may comprise a plurality of unfastened regions (Uᵣ) connected to each other by the discrete bonding regions (Bᵣ), preferably wherein each said unfastened region (Uᵣ) is positioned adjacent to at least one of the discrete bonding regions (Bᵣ). Advantageously, this permits to maximise the surface area of liquid distribution by mass flow during an initial wetting period.

Preferably, the unfastened regions (Uᵣ) are comprised in a plurality (i.e. a plurality of unfastened regions) and separated by discrete bonding regions (Bᵣ), preferably wherein said plurality of unfastened regions (Uᵣ) separated by discrete bonding regions (Bᵣ) are positioned in a front half and/or rear half of the absorbent core (4). The front half of the core may be positioned between a front transverse edge (12') and a transverse centerline (x), and the rear (or back) half of the absorbent core may be positioned between a rear transverse edge (12) and the transverse centerline (x). Advantageously risk of stiffening structures formed at the center of the core are reduced.

Preferably, the discrete bonding regions (Bᵣ) are separated by one or more unfastened regions (Uᵣ) along an axis parallel to a transversal centerline (x) that is substantially perpendicular to the longitudinal centreline (y). Advantageously, this may allow greater absorbent material restriction to movement along the core in the longitudinal direction in front/rear halves of the core towards the center or vice-versa and hence contributing to core integrity. Moreover, this arrangement may aid guiding of absorbent material into the cavity of the unfastened regions during wetting whilst swelling of absorbent material in neighbouring areas within the absorbent deposition zone occurs.

The size (e.g. width-wise dimension) of the unfastened regions (Ur) positioned between discrete bonding regions (Bᵣ) taken along an axis parallel to a transversal centerline (x) may be from about 3mm to about 60mm, preferably from about 5mm to about 40mm, even more preferably from about 10mm to about 30mm.

The unfastened regions (Ur) and the discrete bonding regions (Br) collectively may form one or more channels (9) suitable for guiding liquid via mass flow (for example similar to flow of liquid along a duct) therethrough and/or therealong during a first time period, and wherein one or more portions of said channel(s) corresponding to the unfastened regions (Ur) open (for example top and bottom core wrap layers move further away from each other to increase a cavity volume therebetween) to further accommodate absorbent material (5) therein as adjacent absorbent material (5) of the absorbent-material-deposition zone (Zamd) swells. Advantageously optimised core usage also during subsequent wetting(s) may be attained.

The unfastened regions (Ur) may be suitable for guiding liquid by capillary flow (for example by wicking of liquid between narrow spaces within the absorbent material) at least during a second time period for example once absorbent material enters the cavity formed by said unfastened regions (Ur) during swelling of the absorbent material in neighbouring areas of the absorbent-material-deposition zone. Advantageously this may permit liquid absorption also against gravity and/or movement of the subject especially during an already wetted state.

Preferably the second time period occurs after the first time period; and/or the second time period is greater than the first time period; and/or the second time period is more than 15 seconds, preferably more than 20 seconds (for example from 25 seconds to 2 minutes, preferably from 30 seconds to 1 minute). Advantageously this permits delayed activity of capillary flow after an initial mass flow occurrence such to minimise slowing down of liquid during first wetting along the core.

The top and bottom core wrap layers (7, 8) are preferably joined together by one or more adhesives and/or mechanical bonds. Advantageously this permits not only to attain excellent liquid transfer performance but further also aids to retain core integrity particularly during wetting and swelling of the absorbent material. This may be the case at the bonding region(s) (Br) herein and/or in other regions of the core such as the peripheral edges so as to seal and enclose the absorbent material to prevent lateral outflow thereof.

In an embodiment, as exemplified in Figs. 2 and 6 to 9, the top core wrap layer (7) or bottom core wrap layer (8) comprises one or more stripes of first adhesive (A1) that may be continuous or discontinuous. Preferably, the top core wrap layer (7) or bottom core wrap layer (8) comprises a plurality of, preferably spaced apart, stripes of first adhesive (A1) extending along an axis substantially parallel to the longitudinal centerline (y).

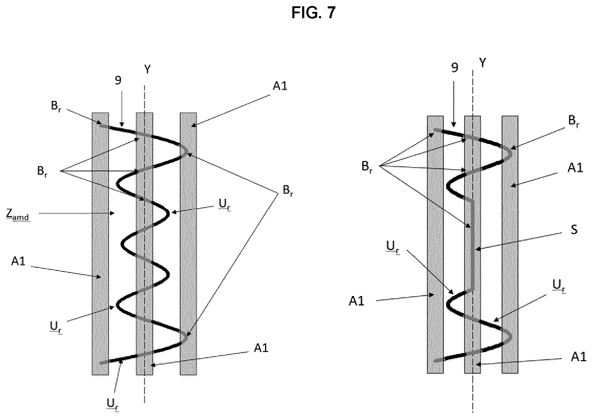

In an embodiment, as exemplified in Figs. 6 and 7, the stripes of adhesive may have an aspect ratio of greater than 1, preferably greater or equal to 1.5, even more preferably from 2 to 160. The stripes generally extending along an axis substantially parallel to the longitudinal centreline (y), and generally wherein at least one, preferably at least two, of said stripes overlaps with one or more, preferably a plurality, of the discrete bonding region(s) (Bᵣ). The stripes being preferably arranged such that each of said stripes does not substantially overlap (for example overlaps less than 20% of its width or area, preferably about 0% of its width or area) with the one or more unfastened regions (Uᵣ), preferably wherein each said stripe overlaps at least a portion of the absorbent-material-deposition zone (Z_{amd}). Advantageously this allows to form bonding region(s) and unfastened regions in an effective manner without compromising production speed and yet provide some resistance to movement of the absorbent material also within the absorbent-material-deposition zone.

Preferably, the opposing bottom core wrap layer (8) or top core wrap layer (7) is substantially free of adhesive at least in areas that are congruent (typically meaning herein "coinciding when superimposed") with the stripes of first adhesive (A1). Advantageously this reduces risk of contamination by residual absorbent material sticking to the bonding region that may impact joining strength and hence also core integrity.

Preferably, as exemplified in Fig. 2, the absorbent core (4) comprises a left longitudinal half (L) and right longitudinal half (R) running along a length of said core (4) and being adjacent to each other with the longitudinal centerline (y) being interposed therebetween, and wherein said plurality of stripes (A1) comprise at least two, preferably spaced apart, stripes that are positioned on said left longitudinal half (L) and/or right longitudinal half (R). Neighbouring (e.g. ones that are closest to each other e.g. directly neighbouring) bonding regions (Bᵣ) are free of unfastened regions (Uᵣ) in direct connection therewith along the longitudinal direction (L_{d}). Advantageously, this may allow avoiding an unfastened region in between and adjacent two consecutive bonding regions in the longitudinal direction and hence permit hinge structures aiding cup formation during wetting to be formed for better comfort as well as increasing area available for absorption.

In an embodiment, example shown in Figs. 6 and 7, at least one of said plurality of stripes, preferably one, overlaps and/or coincides with the longitudinal centerline of the absorbent core (4). Advantageously, this provides for (discrete) bonding regions coinciding with the longitudinal centerline of the absorbent core (4) which would provide for improved core integrity along said centerline.

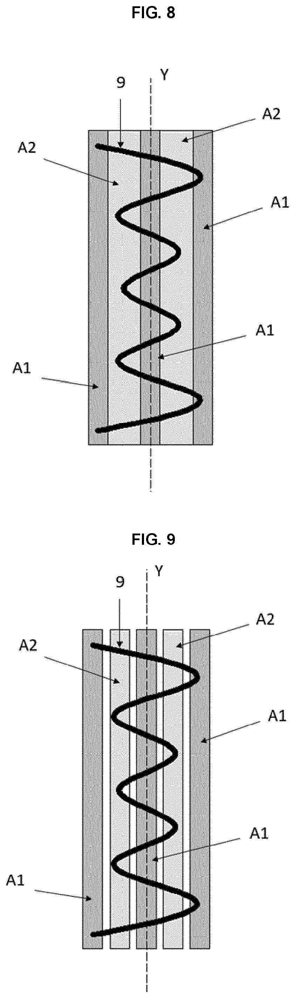

In an embodiment, as shown as example in Fig. 8 and 9, the top core wrap layer (7) or bottom core wrap layer (8) comprises a plurality of, preferably spaced apart, stripes of second adhesive (A2) extending along an axis substantially parallel to the longitudinal centerline (y) having a different basis weight of adhesive than the first adhesive (A1).

In an embodiment, as shown as example in Fig. 8 and 9, the first adhesive (A1) is substantially complementary to the second adhesive (A2) or wherein the stripes of first adhesive are spaced apart from the stripes of second adhesive (A2).

Preferably, the unfastened regions (Uᵣ) and the discrete bonding regions (Bᵣ) collectively correspond to a shape of an insert (sometimes herein referred to as non-porous insert) generally according to equipment and methods described herein-below.

Advantageously this allows to ensure good core integrity as well as maximised surface area coverage of liquid distribution via mass flow over an initial wetting period, yet permit volume expansion within cavities formed in the unfastened regions to accommodate neighbouring absorbent material as it swells whilst minimising the size of restriction areas formed at top/bottom core wrap joints. I may also further aid to reduce cost by permitting a reduction in amount of absorbent material yet retain a larger area for swelling capacity in wet state.

Advantages of the configurations exemplified in Figs. 1-9, include for example the possibility of forming channels via a single and/or continuous insert (as generally described herein-below) which can easily be mounted onto pockets of a rotating drum (as generally described herein-below) and further allows even pressure to be applied by pressure rollers (such as generally described herein-below) that can continuously glide over an evenly elevated surface hence increasing lifespan of components and allowing operation at higher speeds. In contrast, using multiple distinct inserts may not only increase complexity in accurate mounting and changeover time increase, but further included multiple discrete elevated zones so that the pressure roller(s) are forced to bounce up and down, leading to higher variability in the process and greater wear & tear.

### ARRAY OF PACKAGES

In an embodiment, an array of packages is provided comprising at least a first package comprising a first plurality of absorbent articles (1) according to the present invention and at least a second package comprising a second plurality of absorbent articles (1) according to the present invention. The first and second plurality of absorbent articles differ in terms of one or more characteristics selected from: average apex-to-centerline distance (D) in the front portion (F), average apex-to-centerline distance (D) in the crotch portion (C), average apex-to-centerline distance (D) in the rear portion (B). Advantageously, such arrays provide for a range of absorbent articles adapted to differences in the needs of the user. For example, such arrays can provide for a package of articles wherein the one or more meandering channels is adapted to male users and for a package of articles wherein the one or more meandering channels is adapted to female users. In another example, such arrays can provide for a package of articles wherein the one or more meandering channels is adapted to usage during the day and for a package of articles wherein the one or more meandering channels is adapted to usage during the night.

### METHODS

### Average apex-to-centerline distance (D)

The apex-to-centerline distance (D) for a turning point (T) is measured between the longitudinal centerline (y) of the absorbent core (4) and the apex (X) of said turning point (T). This distance is measured from the apex (X) orthogonally to the longitudinal centerline (y), as shown in Fig. 11.

These measurements are performed on 10 absorbent articles (1) after which the average is calculated of the apex-to-centerline distance (D) of all turning points (T). The average apex-to-centerline distance (D) in the front portion (F) is the average calculated for all turning points (T) positioned in said front portion (F) of said 10 absorbent articles (1). Same is valid for the crotch portion (C) and the rear portion (B).

## Claims

1. An absorbent article (1) comprising:
a topsheet (2);
a backsheet (3); and
an absorbent core (4) sandwiched between said topsheet (2) and backsheet (3) and comprising a first longitudinal half and a second longitudinal half separated by a longitudinal centerline (y);
said core (4) comprising absorbent material (5) that is substantially enclosed by a core wrap (6); wherein said core wrap (6) comprises a top core wrap layer (7) and a bottom core wrap layer (8); said core (4) comprising one or more channels (9) flanked by absorbent material (5) that is positioned at a first absorbent material deposition zone (Z_{amd1})
**characterised in that** at least one of the channels (9), preferable each said channel, has a meandering shape extending along a longitudinal direction (Ld) running substantially parallel to a longitudinal centerline (y) of said absorbent core (4) and comprising a plurality of turning points (T); each of said turning points (T) forming an apex (X); wherein the plurality of turning points (T) comprises at least a first turning point (T1, T1') and at least a second turning point (T2, T2'); wherein at least a left first turning point (T1) is located in the first longitudinal half (L) and wherein at least a left second turning point (T2) is located in the first longitudinal half (L); wherein at least a right first turning point (T1') is located in the second longitudinal half (R) and wherein at least a right second turning point (T2') is located in the second longitudinal half (R) and wherein said at least first turning point (T1, T1') is offset from said at least second turning point (T2, T2') such that an imaginary line (I1, I2) running parallel to the longitudinal centerline (y) crosses the apex (X) of the at least first turning point (T1, T1') and not the apex (X) of the at least second turning point (T2, T2') or vice versa.

2. An absorbent article (1) according to Claim 1, wherein an imaginary line (I1, I2) running parallel to the longitudinal centerline (y) crosses the apex (X) of the at least left first turning point (T1) and not the apex (X) of the at least left second turning point (T2) or vice versa; and/or wherein an imaginary line (I1, I2) running parallel to the longitudinal centerline (y) crosses the apex (X) of the at least right first turning point (T1') and not the apex (X) of the at least right second turning (T2') point or vice versa.

3. An absorbent article (1) according to any of the preceding Claims, wherein said meandering shape further comprises a straight section (S) running substantially parallel to a longitudinal centerline (y) of said absorbent core (4).

4. An absorbent article (1) according to any of the preceding Claims, wherein the absorbent core (4) comprises a front portion (F), a crotch portion (C) and a rear portion (B); and wherein the at least one meandering channel (9) extends into the front (F) and/or rear portion (B).

5. An absorbent article (1) according to any of the preceding Claims, wherein the average apex-to-centerline distance (D) is lower in the front portion (F) or rear portion (B) than in the crotch portion (C).

6. An absorbent article (1) according to any of Claims 1-4, wherein the average apex-to-centerline distance (D) is lower in the front portion (F) and rear portion (B) than in the crotch portion (C).

7. An absorbent article (1) according to any of Claims 1-4, wherein the average apex-to-centerline distance (D) is lower in the crotch portion (C) than in the front portion (F) and rear portion (B).

8. An absorbent article (1) according to any of Claims 1-4, wherein the average apex-to-centerline distance (D) is the same in the crotch portion (C) as in the front (F) and rear portions (B).

9. An absorbent article (1) according to any of the preceding Claims, wherein the top core wrap layer (7) is continuously bonded to the bottom core wrap layer (8) along the one or more channels (9).

10. An absorbent article (1) according to any of the preceding Claims, wherein the top core wrap layer (7) or bottom core wrap layer (8) comprises a plurality of, preferably spaced apart, stripes of first adhesive (A1) extending along an axis substantially parallel to the longitudinal centerline (y).

11. An absorbent article (1) according to Claim 10, wherein the opposing bottom core wrap layer (8) or top core wrap layer (7) is free of adhesive at least in areas that are congruent with the stripes of first adhesive (A1).

12. An absorbent article (1) according to any of Claims 10 or 11, wherein the top core wrap layer (7) or bottom core wrap layer (8) comprises a plurality of, preferably spaced apart, stripes of second adhesive (A2) extending along an axis substantially parallel to the longitudinal centerline (y) having a different basis weight of adhesive than the first adhesive (A1).

13. An absorbent article (1) according to Claim 12, wherein the first adhesive (A1) is substantially complementary to the second adhesive (A2) or wherein the stripes of first adhesive (A1) are spaced apart from the stripes of second adhesive (A2).

14. An absorbent article (1) according to any of Claims 10 to 13, wherein the top core wrap layer (7) and the bottom core wrap layer (8) are bonded at one or more bonding regions (Br).

15. An absorbent article (1) according to Claim 14, wherein the one or more bonding regions (Br) comprise permanent and/or temporary attachments.

## Patentansprüche

1. Ein absorbierender Artikel (1), umfassend:
eine Oberschicht (2);
eine Unterschicht (3);
und einen absorbierenden Kern (4), der zwischen der besagten Oberschicht (2) und der besagten Unterschicht (3) sandwichartig angeordnet ist und eine erste Längshälfte sowie eine zweite Längshälfte umfasst, die durch eine Längsmittellinie (y) getrennt sind; wobei der besagte Kern (4) absorbierendes Material (5) umfasst, das im Wesentlichen von einer Kernumhüllung (6) umschlossen ist; wobei die besagte Kernumhüllung (6) eine obere Kernumhüllungsschicht (7) und eine untere Kernumhüllungsschicht (8) umfasst; wobei der besagte Kern (4) einen oder mehrere Kanäle (9) umfasst, die von absorbierendem Material (5) flankiert sind, das in einer ersten Abscheidungszone für absorbierendes Material (Zamd1) positioniert ist,
**dadurch gekennzeichnet, dass** mindestens einer der Kanäle (9), vorzugsweise jeder besagte Kanal, eine mäandernde Form aufweist, die sich entlang einer Längsrichtung (Ld) erstreckt, die im Wesentlichen parallel zu einer Längsmittellinie (y) des besagten absorbierenden Kerns (4) verläuft und eine Vielzahl von Wendepunkten (T) umfasst; wobei jeder der besagten Wendepunkte (T) einen Scheitelpunkt (X) bildet; wobei die Vielzahl von Wendepunkten (T) mindestens einen ersten Wendepunkt (T1, T1') und mindestens einen zweiten Wendepunkt (T2, T2') umfasst; wobei sich mindestens ein linker erster Wendepunkt (T1) in der ersten Längshälfte (L) befindet und wobei sich mindestens ein linker zweiter Wendepunkt (T2) in der ersten Längshälfte (L) befindet; wobei sich mindestens ein rechter erster Wendepunkt (T1') in der zweiten Längshälfte (R) befindet und wobei sich mindestens ein rechter zweiter Wendepunkt (T2') in der zweiten Längshälfte (R) befindet und wobei der besagte mindestens erste Wendepunkt (T1, T1') gegenüber dem besagten mindestens zweiten Wendepunkt (T2, T2') so versetzt ist, dass eine gedachte Linie (I1, I2), die parallel zur Längsmittellinie (y) verläuft, den Scheitelpunkt (X) des mindestens ersten Wendepunkts (T1, T1') kreuzt und nicht den Scheitelpunkt (X) des mindestens zweiten Wendepunkts (T2, T2') oder umgekehrt.

2. Ein absorbierender Artikel (1) nach Anspruch 1, wobei eine gedachte Linie (I1, I2), die parallel zur Längsmittellinie (y) verläuft, den Scheitelpunkt (X) des mindestens linken ersten Wendepunkts (T1) kreuzt und nicht den Scheitelpunkt (X) des mindestens linken zweiten Wendepunkts (T2) oder umgekehrt; und/oder wobei eine gedachte Linie (I1, I2), die parallel zur Längsmittellinie (y) verläuft, den Scheitelpunkt (X) des mindestens rechten ersten Wendepunkts (T1') kreuzt und nicht den Scheitelpunkt (X) des mindestens rechten zweiten Wendepunkts (T2') oder umgekehrt.

3. Ein absorbierender Artikel (1) nach einem der vorhergehenden Ansprüche, wobei die besagte mäandernde Form ferner einen geraden Abschnitt (S) umfasst, der im Wesentlichen parallel zu einer Längsmittellinie (y) des besagten absorbierenden Kerns (4) verläuft.

4. Ein absorbierender Artikel (1) nach einem der vorhergehenden Ansprüche, wobei der absorbierende Kern (4) einen vorderen Bereich (F), einen Schrittbereich (C) und einen hinteren Bereich (B) umfasst; und wobei sich der mindestens eine mäandernde Kanal (9) in den vorderen (F) und/oder hinteren Bereich (B) erstreckt.

5. Ein absorbierender Artikel (1) nach einem der vorhergehenden Ansprüche, wobei der durchschnittliche Abstand vom Scheitelpunkt zur Mittellinie (D) im vorderen Bereich (F) oder im hinteren Bereich (B) geringer ist als im Schrittbereich (C).

6. Ein absorbierender Artikel (1) nach einem der Ansprüche 1-4, wobei der durchschnittliche Abstand vom Scheitelpunkt zur Mittellinie (D) im vorderen Bereich (F) und im hinteren Bereich (B) geringer ist als im Schrittbereich (C).

7. Ein absorbierender Artikel (1) nach einem der Ansprüche 1-4, wobei der durchschnittliche Abstand vom Scheitelpunkt zur Mittellinie (D) im Schrittbereich (C) geringer ist als im vorderen Bereich (F) und im hinteren Bereich (B).

8. Ein absorbierender Artikel (1) nach einem der Ansprüche 1-4, wobei der durchschnittliche Abstand vom Scheitelpunkt zur Mittellinie (D) im Schrittbereich (C) derselbe ist wie im vorderen (F) und hinteren Bereich (B).

9. Ein absorbierender Artikel (1) nach einem der vorhergehenden Ansprüche, wobei die obere Kernumhüllungsschicht (7) entlang des einen oder der mehreren Kanäle (9) durchgehend mit der unteren Kernumhüllungsschicht (8) verbunden ist.

10. Ein absorbierender Artikel (1) nach einem der vorhergehenden Ansprüche, wobei die obere Kernumhüllungsschicht (7) oder die untere Kernumhüllungsschicht (8) eine Vielzahl von, vorzugsweise beabstandeten, Streifen eines ersten Klebstoffs (A1) umfasst, die sich entlang einer Achse erstrecken, die im Wesentlichen parallel zur Längsmittellinie (y) verläuft.

11. Ein absorbierender Artikel (1) nach Anspruch 10, wobei die gegenüberliegende untere Kernumhüllungsschicht (8) oder obere Kernumhüllungsschicht (7) zumindest in Bereichen, die mit den Streifen des ersten Klebstoffs (A1) kongruent sind, frei von Klebstoff ist.

12. Ein absorbierender Artikel (1) nach einem der Ansprüche 10 oder 11, wobei die obere Kernumhüllungsschicht (7) oder die untere Kernumhüllungsschicht (8) eine Vielzahl von, vorzugsweise beabstandeten, Streifen eines zweiten Klebstoffs (A2) umfasst, die sich entlang einer Achse erstrecken, die im Wesentlichen parallel zur Längsmittellinie (y) verläuft und ein anderes Flächengewicht als der erste Klebstoff (A1) aufweisen.

13. Ein absorbierender Artikel (1) nach Anspruch 12, wobei der erste Klebstoff (A1) im Wesentlichen komplementär zum zweiten Klebstoff (A2) ist oder wobei die Streifen des ersten Klebstoffs (A1) von den Streifen des zweiten Klebstoffs (A2) beabstandet sind.

14. Ein absorbierender Artikel (1) nach einem der Ansprüche 10 bis 13, wobei die obere Kernumhüllungsschicht (7) und die untere Kernumhüllungsschicht (8) in einem oder mehreren Verbindungsbereichen (Br) verbunden sind.

15. Ein absorbierender Artikel (1) nach Anspruch 14, wobei der eine oder die mehreren Verbindungsbereiche (Br) permanente und/oder temporäre Befestigungen umfassen.

## Revendications

1. Un article absorbant (1) comprenant :
une feuille de dessus (2) ;
une feuille de fond (3) ;
et un noyau absorbant (4) pris en sandwich entre ladite feuille de dessus (2) et ladite feuille de fond (3) et comprenant une première moitié longitudinale et une seconde moitié longitudinale séparées par une ligne centrale longitudinale (y) ;
ledit noyau (4) comprenant un matériau absorbant (5) qui est sensiblement enfermé par une enveloppe de noyau (6) ; dans lequel ladite enveloppe de noyau (6) comprend une couche d'enveloppe de noyau supérieure (7) et une couche d'enveloppe de noyau inférieure (8) ; ledit noyau (4) comprenant un ou plusieurs canaux (9) flanqués par du matériau absorbant (5) qui est positionné au niveau d'une première zone de dépôt de matériau absorbant (Zamd1)
**caractérisé en ce que** au moins un des canaux (9), de préférence chaque dit canal, a une forme sinueuse s'étendant le long d'une direction longitudinale (Ld) s'étendant sensiblement parallèlement à une ligne centrale longitudinale (y) dudit noyau absorbant (4) et comprenant une pluralité de points de retournement (T) ; chacun desdits points de retournement (T) formant un sommet (X) ; dans lequel la pluralité de points de retournement (T) comprend au moins un premier point de retournement (T1, T1') et au moins un second point de retournement (T2, T2') ; dans lequel au moins un premier point de retournement gauche (T1) est situé dans la première moitié longitudinale (L) et dans lequel au moins un second point de retournement gauche (T2) est situé dans la première moitié longitudinale (L) ; dans lequel au moins un premier point de retournement droit (T1') est situé dans la seconde moitié longitudinale (R) et dans lequel au moins un second point de retournement droit (T2') est situé dans la seconde moitié longitudinale (R) et dans lequel ledit au moins premier point de retournement (T1, T1') est décalé par rapport audit au moins second point de retournement (T2, T2') de telle sorte qu'une ligne imaginaire (I1, I2) s'étendant parallèlement à la ligne centrale longitudinale (y) croise le sommet (X) de l'au moins premier point de retournement (T1, T1') et non le sommet (X) de l'au moins second point de retournement (T2, T2') ou vice versa.

2. Un article absorbant (1) selon la revendication 1, dans lequel une ligne imaginaire (I1, I2) s'étendant parallèlement à la ligne centrale longitudinale (y) croise le sommet (X) de l'au moins premier point de retournement gauche (T1) et non le sommet (X) de l'au moins second point de retournement gauche (T2) ou vice versa ; et/ou dans lequel une ligne imaginaire (I1, I2) s'étendant parallèlement à la ligne centrale longitudinale (y) croise le sommet (X) de l'au moins premier point de retournement droit (T1') et non le sommet (X) de l'au moins second point de retournement droit (T2') ou vice versa.

3. Un article absorbant (1) selon l'une quelconque des revendications précédentes, dans lequel ladite forme sinueuse comprend en outre une section droite (S) s'étendant sensiblement parallèlement à une ligne centrale longitudinale (y) dudit noyau absorbant (4).

4. Un article absorbant (1) selon l'une quelconque des revendications précédentes, dans lequel le noyau absorbant (4) comprend une partie avant (F), une partie d'entrejambe (C) et une partie arrière (B) ; et dans lequel l'au moins un canal sinueux (9) s'étend dans la partie avant (F) et/ou la partie arrière (B).

5. Un article absorbant (1) selon l'une quelconque des revendications précédentes, dans lequel la distance moyenne du sommet à la ligne centrale (D) est plus faible dans la partie avant (F) ou la partie arrière (B) que dans la partie d'entrejambe (C).

6. Un article absorbant (1) selon l'une quelconque des revendications 1 à 4, dans lequel la distance moyenne du sommet à la ligne centrale (D) est plus faible dans la partie avant (F) et la partie arrière (B) que dans la partie d'entrejambe (C).

7. Un article absorbant (1) selon l'une quelconque des revendications 1 à 4, dans lequel la distance moyenne du sommet à la ligne centrale (D) est plus faible dans la partie d'entrejambe (C) que dans la partie avant (F) et la partie arrière (B).

8. Un article absorbant (1) selon l'une quelconque des revendications 1 à 4, dans lequel la distance moyenne du sommet à la ligne centrale (D) est la même dans la partie d'entrejambe (C) que dans les parties avant (F) et arrière (B).

9. Un article absorbant (1) selon l'une quelconque des revendications précédentes, dans lequel la couche d'enveloppe de noyau supérieure (7) est liée en continu à la couche d'enveloppe de noyau inférieure (8) le long du ou des canaux (9).

10. Un article absorbant (1) selon l'une quelconque des revendications précédentes, dans lequel la couche d'enveloppe de noyau supérieure (7) ou la couche d'enveloppe de noyau inférieure (8) comprend une pluralité de bandes, de préférence espacées, de premier adhésif (A1) s'étendant le long d'un axe sensiblement parallèle à la ligne centrale longitudinale (y).

11. Un article absorbant (1) selon la revendication 10, dans lequel la couche d'enveloppe de noyau inférieure opposée (8) ou la couche d'enveloppe de noyau supérieure (7) est exempte d'adhésif au moins dans les zones qui sont congruentes avec les bandes de premier adhésif (A1).

12. Un article absorbant (1) selon l'une quelconque des revendications 10 ou 11, dans lequel la couche d'enveloppe de noyau supérieure (7) ou la couche d'enveloppe de noyau inférieure (8) comprend une pluralité de bandes, de préférence espacées, de second adhésif (A2) s'étendant le long d'un axe sensiblement parallèle à la ligne centrale longitudinale (y) ayant un grammage d'adhésif différent de celui du premier adhésif (A1).

13. Un article absorbant (1) selon la revendication 12, dans lequel le premier adhésif (A1) est sensiblement complémentaire au second adhésif (A2) ou dans lequel les bandes de premier adhésif (A1) sont espacées des bandes de second adhésif (A2).

14. Un article absorbant (1) selon l'une quelconque des revendications 10 à 13, dans lequel la couche d'enveloppe de noyau supérieure (7) et la couche d'enveloppe de noyau inférieure (8) sont liées au niveau d'une ou plusieurs régions de liaison (Br).

15. Un article absorbant (1) selon la revendication 14, dans lequel la ou les régions de liaison (Br) comprennent des attaches permanentes et/ou temporaires.
